# EUROPEAN PATENT SPECIFICATION

(11) **EP 2 127 518 B1**
(45) Date of publication and mention of the grant of the patent: **04.11.2015**
(21) Application number: 09161447.9
(22) Date of filing: 28.05.2009
(51) Int. Cl.: B08B 7/00, A01K 75/00

(54) **Floating device to clean nets**
Schwimmvorrichtung zum Reinigen von Netzen
Dispositif flottant pour nettoyer les filets

(30) Priority: 29.05.2008 CL 15652008
(43) Date of publication of application: 02.12.2009
(73) Proprietor: Ochoa Disselkoen, Jose Alberto, Macul, Santiago (CL)
(72) Inventor: Ochoa Disselkoen, Jose Alberto, Macul, Santiago (CL)
(74) Representative: Lorente Berges, Ana

(56) References cited:
- WO-A-2008/026933
- FR-A- 2 862 276
- JP-A- 11 278 374
- JP-A- 2004 081 119
- JP-U- 1 082 761

## Description

This invention relates to a submergible and floating device for the on-site biological cleansing of nets or meshes used in fish breeding operations, more specifically, the device includes means that allow the retention of particles and sources of radiation (UV) that permit the destruction of microorganisms in the water and at the same time disinfect and destroy bacteria, fungi, viruses and fish diseases that might otherwise spread to the breeding medium.

In order to understand with greater clarity the technical solution provided by the invention, we will describe the means and methods currently in use for cleaning and maintenance and the materials used for nets or meshes used in fish-breeding cages.

In mid spring, the temperature of the water increases and with it its luminosity, generating a sharp increase in algae growth and the spawning of bivalve molluscs, baianus, piures (Pyura chilensis), sea lice and other species that adhere to the nets that comprise the walls of the cages for the fattening of salmon and sea trout. These colonies are often so abundant that they block a substantial part of the exchange of water between the cages and the environment. The oxygen content of the internal waters drops causing a strong stress, a diminution in growth and even a rise in mortality, which affects the profitability of the salmon fisheries industry. This is particularly so because this phenomenon is accentuated during the summer, causing the death of fish in the harvesting stage. Normally the nets are changed in a task that requires divers and specialized personnel. The changeover of nets is a risky business as fish usually escape and also because the tide frequently closes the bags formed by the nets that are being changed, asphyxiating many fish. The complexity of the operation and the need to use divers, specialized staff, boats, etc., make it not only a risky operation but a costly one too, from an operational point of view. Once on land, the nets are washed using pressurized fresh water, they are dried and then impregnated with antifouling liquid, after which they are ready to be used again. When the rise in water temperature and luminosity is sudden, or the harvesting is postponed, and the lack of oxygen in the water inside the cages becomes critical, there is an emergency situation. The emergency must be resolved using extraordinary means and this is the in situ cleansing of the nets using a machine handled by divers that scrapes off the adhered matter, sucking it up. All the resulting organic material is poured into the ocean, causing a great level of contamination. This measure, which is forbidden under the rules and regulations of the environmental, fisheries, maritime and sanitary authorities, is only used in the event of an emergency. In situ cleansing of the nets by means of a technology authorized by said environmental, fisheries, maritime and sanitary authorities would significantly lower production costs, together with lowering fish mortality rates, both of which are factors with a strong incidence on the profitability of the salmon business.

There are two scenarios in which the nets operate in the salmon industry, both of which are directly related to the two essential phases of the intensive breeding of salmon: the Freshwater Phase and the Marine Phase.

In the Freshwater Phase one can frequently see salmon in the smolting stage in fish pens installed in lakes or the estuaries of rivers. In the marine or fattening stage, which is the final stage of the breeding process, the fish are kept under strict control in cages whose structures are generally either square or round, although there are also, octagonal and polygonal cages, which consist of a structure that can be made of hot-galvanized iron or high-density polystyrene with expanded polystyrene or PVC floats and a net consisting of nylon or polyester sheets that contain the fish.

In order to install the fish pens, weights are placed on the bottom-side corners of the net, in order to maintain their form and anchoring or moorings are fitted to the floating structure to keep the fish pens in place. In spite of this, this anchoring system must allow for a certain flexibility of movement, as these systems are subject to the effects of currents and tides. Also, a net is placed rising to a height of approximately one meter over the cage to prevent the escape of salmon and depredation by marine bird species. The most frequently used dimensions for the fish pens are 20 x 20 x 17 to 30 x 30 x 17 for square shaped version, and thirty meters in diameter by 17 metres depth for circular units.

During the marine phase, the nets that make up the fish rearing pens need to be changed for nets with larger net openings as the fish increase in size and calibre.

For smolt that is just being brought into a saltwater environment, nets with 1,27 cm (½") openings are used for a period of two months. Subsequently these are replaced by 2,54 cm (1") nets for a period of 6 months and finally, the net is changed for one last time, replacing it with a 3,81 cm (1.5") to 5,08 cm (2") net until the harvesting period. These nets also need to be changed periodically due to the marine incrustations phenomenon that affects them, as the nets must remain clean to allow for the free circulation of water, which is vital to achieve adequate fish growth. During the summer period, these nets must be changed every fifteen days and during winter, every two months approximately. The above notwithstanding, the application of antifouling paint to the nets makes it possible to prolong the net changeover periods for every three to four months.

In addition to the nets used specifically in the breeding of fish, a perimeter-wide fencing using the so called "predator nets" (loberas) needs to be installed in order to prevent approaches by seals who can obviously break through the nets releasing massive numbers of fish with the resulting economic damages. These nets are very long and 5,500 m2 of netting is used for circular fish pens. Square fish pens, which are usually placed side-by-side like a train, use 12,000 m2 of netting. These "predator nets" usually have a net opening size of 25,4 cm (10") and must also be impregnated with antifouling paint to achieve their maximum permanence in seawater without marine incrustations.

Change of Nets
This operation involves the replacement of a net in use that is "dirty" with incrustations (fouling), algae, invertebrates (crustaceans, molluscs, nematodes, briozoans, annelids) that adhere to and colonize the nets holding the fish in the fattening stage - obstructing the renewal of the water and increasing their weight, putting the buoys and the stability of the fish pens at risk; changing it for a clean net that has been repaired, impregnated and sanitized in order to maintain optimum breeding conditions until the time of harvesting.

Net Cleansing and Washing
This part of the process consists of the removal of algae, molluscs, crustaceans and antifouling paint from the dirty nets of the breeding facilities.

The first stage is done by hand by men out in the open (to remove large scale residues) and then continues using a motor-pump, a mechanical washing machine (continuously or in batches), a high-pressure hydro-washer or inside a rotating drum. This stage implies that the RIL of this process will necessarily involve a high organic load that needs to be treated before disposal. However, the big problem in this stage is that the mud separated from the nets is not treated adequately.

In those net workshops that have treatment plants, solid waste is separated from the liquid component (using solid separation technologies) into (fine and coarse) as well as the decanting and pressing of the fine solids (the filter-press cake permits lowering the levels of humidity and consequently the volumes) as well as storing the coarse solids for pressing at a specially conditioned place prior to their disposal at an authorized dumping area. This is a good practice found only in certain net workshops. Regarding this, this waste must not be regarded as hazardous.

Drying of the Nets The nets that have already been washed may be hung out to dry at ambient temperature or else dried artificially through the application of heat produced by the combustion of some source of energy in drying racks (vertically or horizontally) with forced air circulation via mechanical ventilators.

Net Repairs This follows the drying of the nets and is carried out whenever there are any faults and tears in the nets, sewn with the same type of thread or adding a new sheet or hawser of the same characteristics.

Net Impregnation This stage of the process consists of the application of antifouling paint, impregnating the nets by immersing them in the paint for a period of approximately fifteen minutes. Generally 0.8 and 0.6 litres of paint are used for every kilo of dry netting. Specialists recommend the use of paint without solvents and water-based as these are less polluting.

Pacing and Dispatch In this stage of the process, before the paint dries on the impregnated nets, they are wrapped in plastic sheeting and dispatched to the various breeding centres.

Sanitation of the Nets Optionally, after the washing process, the nets can be pasteurized to ensure their sanitary quality.

Icthiosanitary Risks The transportation of nets and breeding gear from their place of origin to the cleaning workshops involves a certain risk of carrying diseases to new sites, expanding the range of action of such diseases. Given that at present there are no antecedents concerning the sanitary consequences of the activities realized in the framework of the gathering, transportation, washing and impregnation of nets, at the same time that their role in the epidemiology of the diseases is unknown, this is not a minor subject upon evaluating this variable as a source of environmental and economic impact within the industry. In the joint study carried out by Fundacion Chile and the University of Concepcion which included amongst its objectives the detection and identification of pathogens affecting fish that are of sanitary importance in the national salmon industry, on the basis of samples of fouling (embeddings and incrustations), liquid and solid waste, nets both pre- and post-washing coming from workshops in the X Region. During the project, two sanitary sampling campaigns were carried out, involving different washing and impregnation companies. Different types of samples were taken, which were classified as fouling, nets (dirty and clean nets) and liquid residues (raw and percolated industrial residues from the truck). The diagnostic techniques employed for the detection of pathogens were indirect immunofluorescence for the detection of Piscirickettsia saimonis (causal agent of Piscirickettsiosis) and Renibacterium salmoninarum (causal agent of renibacteriosis) and the cellular culture on the CHSE-215 lines (Chinook Salmon Embryo) for the detection of the virus of infectious pancreatic necrosis (IPN). Based on the results of the study it was possible to establish that no Renibacterium salmoninarum, Piscirickettsia salmonis or IPN were detected on the samples of fouling and nets analyzed. However, P. Salmonis was detected in the liquid and solid waste samples from the washing processes taken directly at the outlet of the same.

Public Health Risks At least for the past seven years, different groups, environmental associations, citizens' groups, non industrialized fishermen's unions and bivalve producers have been raising the alarm about the risks associated with the transportation of nets from remote zones with dinoflagellates associated with red tide events, which produce neurotoxins that are deadly for the human being upon consumption of such products.

During the month of April of 2002, in light of the evident red tide contamination of consumers, the doctors at the emergency medical services alerted public opinion about the event. It was assumed that the contamination of areas that were free of these dinoflagellates was caused by the transportation of dirty nets containing filtrators (mussels) from areas in Aysen en route to Puerto Montt or Chiloe for net cleansing.

Later it was demonstrated, upon stopping a ship coming from the X region, that it carried nets from salmon breeding raft-cages with molluscs adhered that had a high level of paralyzing toxin for every 100 micrograms of bivalve flesh. The National Health Service in Llanquihue examined the nets and detected mussels with 12,700 milligrams of paralyzing toxin for every 100 micrograms of bivalve flesh.

It is not one hundred percent clear nor has it been proven that the Salmon industry is responsible for the contamination of areas that are free of red tide given that it has been proven that the drain water from all ships can and does carry countless types of organisms that can colonize new environments. But this is not a new subject, as evidenced by the fact that the main maritime routes and sectors adjoining ports are affected by this type of event. Antofagasta has experienced events as potent as those of the XII Region and yet there is no salmon breeding industry in that coastal area. Besides, this is a worldwide problem.

However, in light of this probability and the spreading and colonizing of the far south by the salmon breeding industry, we cannot overlook this aspect, because maintaining the status of optimum quality of the water in the XI and XII regions must be maintained through the simple measure of banning the transport of nets between producing regions so as not to convert the nets into vectors of diseases, a situation that has a massive impact on production costs.

Antifouling Paints Antifouling paint is a specially conceived paint destined to prevent the adherence of algae and molluscs onto surfaces submerged below the surface in seawater, mainly through their biocide action at a cellular level, as its most frequent use is in boats and aquiculture nets. At present, aquiculture worldwide is utilizing copper-based antifouling paint, which is a product formulated on the basis of cuprous oxide (bio-accumulative chemical compound, algaecide and biocide) and highly toxic solvents, such as ethylbenzene and xylene. The permanence of this product on the painted surfaces depends on various factors such as: zone, seasonality, water temperature, salinity, light, oxygen, and marine currents; therefore its durability can range from a couple of weeks and up to six to eight months on aquiculture nets.

Notwithstanding the fact that copper-based antifouling paints are being used, in the early days of aquiculture in Chile tar was used as antifouling element in nets. There are, also, other alternative antifouling paints such as tributyltin oxide, which is intensely used in the painting of ships and boats of all kinds, but this type of paint is being banned in most developed countries because they consider that it has a high biocide bio-cumulative level in many aquatic organisms. Due to the high impact on the environment and human health that the use of antifouling paint has, the scientific world is developing an area of research which is the search for alternatives that are less harmful to the environment. We can mention among these the use of polymers, enzymes and antifouling products based on natural products.

New Antifouling Technologies The first nets used in the breeding of salmon in raft-cages in the 80s originating in extractive or industrial fishing. Consequently, the material from which they were made did not take into account that these nets would be permanently underwater for 12 months or more, which would therefore allow for the adherence and incrustation of living organisms (fouling) such as algae, mussels, and clams, among others. The net systems that the salmon industry uses aim to perform two actions: the first is to contain the fish in captivity and the second, provide protection against predators ("predator nets"). In these nets, fouling generates several problems for salmon breeding, such as: Obstruction of the circulation of the water and diminution of the oxygen available to the fish and also the possibility of becoming a reservoir for infectious agents causing diseases as well as the increase in weight of the nets, which could tear and allow fish to escape.

A polyamide salmon net weighs between 400 and 600 kilos in the water, whereas a maintenance net can weigh up to 10 tonnes. For this reason it became necessary to impregnate the nets used in the fattening of fish in seawater using copper-based antifouling nets that release cations of this metal that prevent the growth of such algae and other organisms. The durability of this protection depends on the season, and the variables characteristic of each breeding centre, but on average, they must be replaced every three months during the summer and every five months during winter. This task involves major logistics aspects if you consider that in 2004 there were between 70 and 80 million square meters of nets being used by the salmon fisheries.

This fact, together with the environmental problems caused by the systems used to wash these nets has given rise to a series of investigations aiming to diminish or annul incrustations. The following is a breakdown of the most relevant technological advances.

One research project, a 30-month long investigation, has funding in excess of 400 million pesos, distributed among contributions made by FONDEF ( Ch$ 224 million), the University of concepción (Ch$ 101 million 430 thousand), Intesal (Ch$ 15 million 825 thousand) Cultivos ecofish (Ch$ 48 million 300 thousand), Aqua cards (Ch$ 22 million,325 thousand) and Nisa redes (Ch$ 31 million 475 thousand).

The project, titled Prototypes of quitosane and quitosane-copper for nets used in systems for the intensive breeding of fish and its applications as antimicotic agent, in fresh water and antifouling agent for slow release into seawater. The project is directed by the Department of Polymers of the Faculty of Chemical Sciences of the University of Concepción.

Quitosane is a natural polymer obtained from the shells of crustaceans such as crabs (cancer spp), prawn (Pannaeus vannamei), lobster (Palinuridae spp) and krill (Euphasia superb), among others.

Following the successful utilization of quitosane in the development of a film that assists in the treatment of burns and of a controlled-release pesticide, the project has proposed the development of a new application for this natural polymer. Together with a group of companies it is working since December on a project in which rigid and semi-rigid nets emerge as an option, but such technology requires an adaptation to local circumstances. The advantage of traditional nylon nets is the fact that they are ductile and easy to move about, they cost less than the rigid and semi-rigid nets available in the market today, which allows for the use of different sizes of nets depending on the size of the fish. Also, they are easy to build, repair and transport. However, they also have some negative aspects, such as a limited useful life (depending on the characteristics of the material selected and how the fish pen is treated). The nets built with this material are affected by currents and tides; therefore, depending on the site of the breeding facility the net is affected to a lower or higher degree, in detriment of the welfare of the fish. In the case of plastic semi-rigid nets, they are highly durable in time (approximately 7 years); they keep their shape and the volume of water inside the cage benefitting the welfare of the fish and do not require impregnation with antifouling products. Also, even those organisms that do adhere to them are easy to remove from the net. Among its disadvantages there is its lower cost with respect to the knitted net and a more complicated process of installation and manipulation in relation to traditional nets. Finally, rigid metal nets, just like plastic nets, are highly durable, they maintain the volume of the net and, depending on the material they are made of they may act against fouling at sea. However, its disadvantages-additionally to the higher costs and greater difficulty of installation and manipulation, just like in the case of semi-rigid nets has to do with the release of fish and their impact on the environment. Depending on the material used in their construction, they may cause alterations to the aquatic environment due to lixiviation (phenomenon that involves the displacement of soluble substances dispersed in the aquatic medium caused by the movement of the water on the ocean floor) of the material of which the material is made. In this same line of reasoning, in the case of rupture due to natural causes, such as storms for example, the consequences can be much more severe than in the case of a polyamide net.

Last year, a project funded by Fondef sought to produce paints on the basis of quitosane destined for the salmon fisheries. These paints are expected to resolve two of the main problems faced by this sector: the development of fungi in intensive breeding systems (ponds, pens) in sweet water and incrustations (fouling) by bacteria, algae, invertebrates (crustaceans, molluscs, nematodes, bryozoans, annelides) that adhere and colonize the nets containing the fish- in the seawater conditioning stage - obstructing the renewal of the water.

The battle against fungi in breeding systems - fungi that result of the confinement and death of smolt-is being fought using chemical baths of malachite green, formalin, common salt and Pyceze (a product developed by Novartis). The first is prohibited and just like the second can be detected at trace level, which might affect sales; the two remaining products, which are authorized forms of treatment, although they have not been evaluated from the point of view of the environment, involve major costs in the freshwater production stage. The research aims to take advantage of the antimicotic properties of quitosane, using it in paints in order to attack the problem with a natural and environmentally safe product with the added bonus of lower costs.

New Materials for Nets
Nets built using non traditional materials:
Copper meshes
Rigid and semi-rigid plastic meshes
Rigid and semi-rigid metallic meshes.

Copper Meshes
As regards the experience with the use of copper meshes, Codelco Chile, together with the Japanese Company Sambo Copper Atoy Co. Ltd., and Ashimori Industry Co. Ltd., supported by INTESAL are developing a project that focuses on the use of the copper net known as "UR30 Alloy" on the meshes of the raft-cages for salmon fisheries. Early in 2005 the experimental phase began in five breeding centres belonging to companies associated with SalmonChile. According to the existing information, the proposed use of copper in salmon fisheries would be the first opportunity to provide internal demand for this product in Chile, linking two key sectors of Chilean exports.

Copper - Salmon
The test nets, (1 x 1 metres) were installed in February of 2006, in order to test the performance of the material in the oceanographic and operational conditions of Chilean fisheries. An experiment at productive level will be carried out in 2007.

The antifouling power of copper permits the diminishing of maintenance costs derived from the cleaning of nets and the stress suffered by the fish upon reducing the need for handling, achieving lower infrastructure costs as the installation can be recycled once its useful life at sea has ended.

This product is already at an experimental stage in Japan, and although it is around 40% more expensive than the one currently in use, it has a longer useful life - between three and five years -and does not require cleaning sessions every two months. They are simply left in place until their period concludes and then they may be recycled.

A commercial stage necessitates a revision of the weight of these nets to verify whether the flotation capacity of the fish pens can resist that weight. At this point it will be important to define whether it is necessary to develop new models of fish pens. A study carried out by the company INTESAL in 2005 realized a mechanical and structural evaluation of the copper-based nets indicating that in terms of results, the metallic net achieves 3.33 greater resistance to traction than a conventional net but has a weight per area unit 34.66 higher than a traditional net. For this reason, new systems to install and remove these nets from the cages must be conceived as well as a careful design of the different activities during these operations.

Semi- Rigid Nets
On the other hand, there has been an increase in seal attacks aimed at the cages, proving that the "predator net" protection is not always effective. These nets are made using a net known as "braided." According to the calculations of the FIP 2003-32 study, "Interference of Marine Mammals in Fisheries and Aquiculture activities" prepared by Universidad de Valparaiso, Universidad Arturo Prat and Universidad Catolica del Norte, it is estimated that the total cost of the interaction between seals and the salmon fisheries in 2003 amounted to US$ 77 million. These costs have been calculated considering, on the one hand the loss of investment and for the other part the investment in and cost of "predator nets." In the particular case of rigid and semi-rigid nets, it is estimated that they may be a very good alternative to prevent predator attacks and that their massive use could diminish the handling of the fish at the breeding centres. The company Patagonia Salmon Farming is experimenting with zinc coated metallic nets and since mid 2006 with copper alloy pens. To date it has been seen that rigid or semi-rigid meshes function very well as defence against predators. They remain far from the salmon and the nets don't affect or damage the exterior of the fish (loss of scales). Therefore, it is concluded that these nets are safe and resistant. Another relevant fact is that according to observations made during this period these nets don't suffer from major deformations caused by the currents, maintaining more constant volumes for the fish.

However, this change is not going to be so fast or so widespread, as the supply capacity of the providers is not sufficient to absorb the full demand of the industry. Also, rigid and semi-rigid nets are going to be useful only for certain places, where oceanographic and logistics conditions justify their use, given that their cost is higher than that of traditional nets. For example, at the breeding centres of the XII Region, where the transportation of nets to authorized facilities in the regions of Aysen or Los Lagos imply a greater cost in transportation and a higher environmental impact.

The fact that polyamide surfaces offer a greater surface where fouling may adhere complicates the achievement of optimum breeding conditions.

Furthermore, document FR-A-2862276 discloses a submergible and floating device for the on-site biological cleansing of nets according to the preamble of independent claim 1.

In order to solve all the inconveniences there are today, a device according to the invention has been created to permit on site cleaning of the nets, which comprises the technical features of independent claim 1. The filtration element permits the retaining of the contaminating particles and the UV radiation element destroys the smaller particles as well as the microorganisms existing in the water and derived of the on-site cleaning of the nets, returning it to the environment in acceptable conditions according to prevailing standards. It also destroys bacteria, fungi and viruses of fish diseases that would otherwise spread into the environment.

The advantages of the device of the present invention are the following:
- Due to its structural properties, it allows for the on-site cleaning of the nets, avoiding the de-rigging of the nets.
- The device permits an adequate treatment of the sludge removed from the nets, which does not occur in the existing art.
- It permits the disinfection of both the particles that fall off the nets as well as the water where the fish are being bred.
- The device does not obstruct water circulation as occurs at present. On the contrary, it increases the oxygen available to the fish.
- Maintenance costs are considerably lower.

For added clarity of the present invention, it will be described on the basis of the accompanying drawings, without limiting any obvious modifications that may emerge, where:
Figure 1 shows a top view of the device of the invention
Figure 2 shows a front sectional view of the device of the invention
Figure 3 a side sectional view of the device of the invention

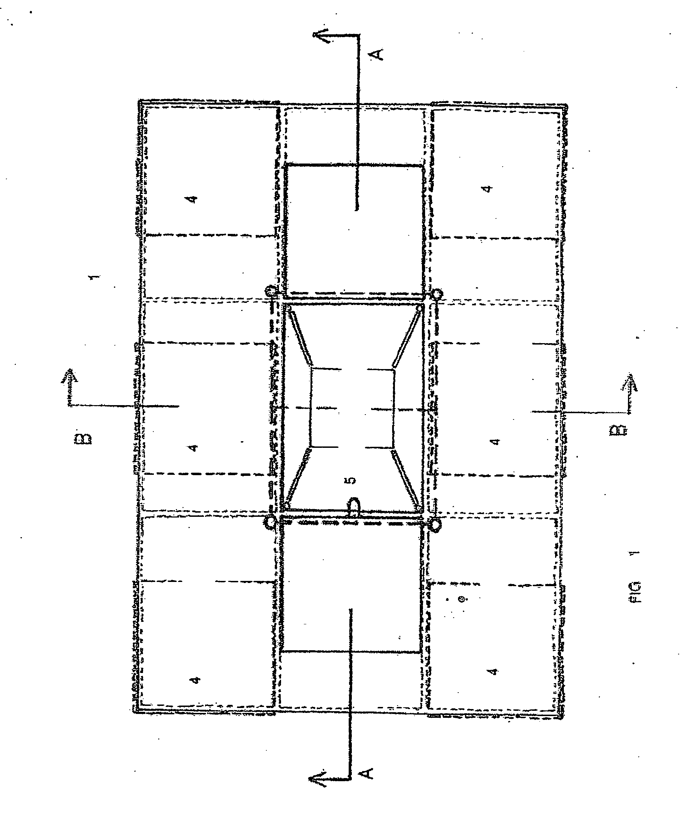

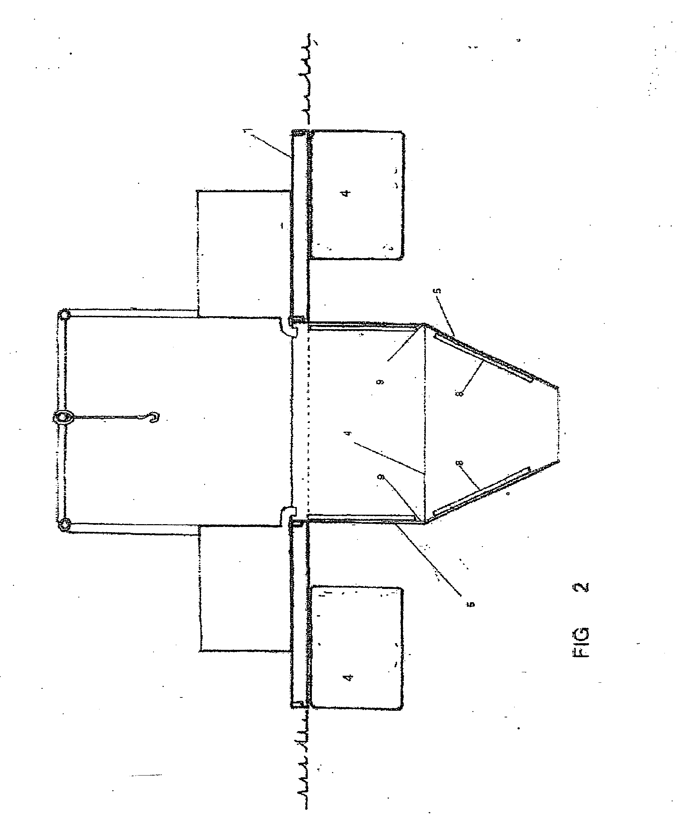

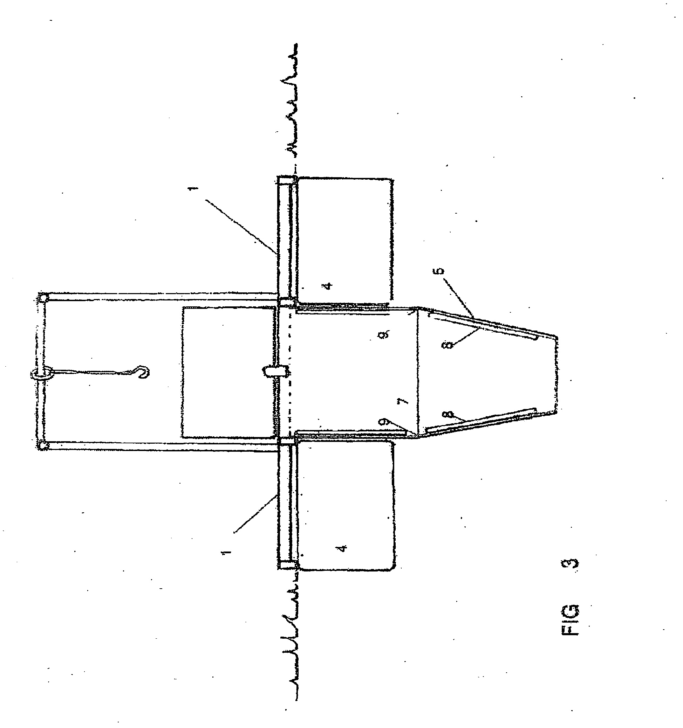

As shown in Figures 1 to 3, the device of the invention for the on-site cleaning of nets or meshes and at the same time the disinfection of the water of the fish breeding facility consists of the supporting means (1), a means to filter particles (2) and a disinfection means based on ultraviolet biological radiation (3).

The supporting means (1) consists of a metal, plastic or wooden structure, with means of flotation (4) which maintain the supporting structure (1) afloat and with a main body that is hollow (5) inside of which are the particle filtering means (2) which consist of a plurality of sacks made of diamond-shaped netting surrounded on the sides and bottom by a polypropylene bolt rope.

The sacks made of diamond-shaped netting consist of a variety of diamond-shaped meshes whose openings are increasingly smaller.

The sacks made of diamond-shaped netting may consist of a variety of diamond-shaped meshes whose openings have increasingly smaller diameters until reaching a mesh with the minimum opening diameter so as to retain and effectively hold all coarse contaminant particles, generally consisting of algae and remains of multi-cellular animals.

The biological radiation means (3) may be provided inside the main hollow body (5) where an ultraviolet radiation chamber is formed that is equipped with a plurality of UV radiation lamps (8) whose potency and number will depend on the flow of liquid and solid waste received. Generally speaking, the greater the flow, the larger the number of lamps and the higher their power, UV radiation increases according to the flow.

The inner walls of the main hollow body (5) may include mirrors (9) which permit the heightening of the effect of the radiation of the said UV radiation lamps (8).

Both the mirrors (9) and the UV radiation lamps (8) may be mounted on the inside of the main hollow body (5) which in turn is supported by the main structure.

## Claims

1. A submergible and floating device for the on-site biological cleansing of nets or meshes used for the breeding of fish, that permits the destruction of the microorganisms of the water and at the same time disinfects and destroys bacteria, fungi, viruses and fish diseases that might otherwise spread onto the breeding environment comprising supporting means (1), a means to filter particles and disinfection means based on ultraviolet biological radiation,
**characterized by** the fact that the supporting means (1) consists of a metal, plastic or wooden structure, which includes flotation means (4) which keep the supporting structure (1) afloat and a main hollow body (5) inside of which are the means to filter particles which consist of a plurality of sacks made of diamond-shaped netting surrounded on the sides and bottom by a polypropylene bolt rope.

2. A submergible and floating device for the on-site biological cleansing of nets or meshes used for the breeding of fish, according to claim 1, **characterized by** the fact that the sacks made of diamond-shaped netting consist of a variety of diamond-shaped meshes whose openings have increasingly smaller diameters until reaching a mesh with the minimum opening diameter so as to retain and effectively hold all coarse contaminant particles, generally consisting of algae and remains of multi-cellular animals.

3. A submergible and floating device for the on-site biological cleansing of nets or meshes used for the breeding of fish, according to cflaim 2, **characterized by** the fact that the biological radiation means (3) is inside the main hollow body (5) where an ultraviolet radiation chamber is formed that is equipped with a plurality of UV radiation lamps (8) whose potency and number will depend on the flow of liquid and solid waste received.

4. A submergible and floating device for the on-site biological cleansing of nets or meshes used for the breeding of fish, according to claim 3, **characterized by** the fact that the inner walls of the main hollow body (5) include mirrors (9) which permit the heightening of the effect of the radiation of the said UV radiation lamps (8).

5. A submergible and floating device for the on-site biological cleansing of nets or meshes used for the breeding of fish, according to claim 4, **characterized by** the fact that both the mirrors (9) and the UV radiation lamps (8) are mounted on the inside of the main hollow body (5) which in turn is supported by the main structure.

## Patentansprüche

1. Tauchfähige und schwimmende Vorrichtung für die biologische Reinigung vor Ort von Netzen oder Geflechten, die zur Fischzucht verwendet werden, die die Zerstörung der Mikroorganismen des Wassers ermöglicht und gleichzeitig Bakterien, Pilze, Viren und Fischkrankheiten desinfiziert und zerstört, die sich andernfalls auf die Zuchtumgebung ausbreiten könnten, aufweisend ein Trägermittel (1), ein Mittel zum Filtern von Teilchen und ein Desinfektionsmittel, das auf einer biologischen Ultraviolettstrahlung beruht,
**gekennzeichnet durch** die Tatsache, dass das Trägermittel (1) aus einer Metall-, Kunststoff- oder hölzernen Struktur besteht, die Schwimmer (4) enthält, die die Trägerstruktur (1) über Wasser halten, und einen Haupthohlkörper (5), in dessen Inneren sich die Mittel zum Filtern von Teilchen befinden, die aus mehreren Säcken bestehen, die aus einem rautenförmigen Netzwerk gemacht sind, das an den Seiten und am Boden von einem Polypropylenliektau umgeben ist.

2. Tauchfähige und schwimmende Vorrichtung für die biologische Reinigung vor Ort von Netzen oder Geflechten, die zur Fischzucht verwendet werden, nach Anspruch 1, **gekennzeichnet durch** die Tatsache, dass die Säcke, die aus einem rautenförmigen Netzwerk gemacht sind, aus einer Vielzahl von rautenförmigen Maschen bestehen, deren Öffnungen zunehmend kleinere Durchmesser aufweisen, bis eine Masche mit dem minimalen Öffnungsdurchmesser erreicht ist, so dass alle groben Verunreinigungsteilchen zurückgehalten und effektiv gehalten werden, die im Allgemeinen aus Algen und Rückständen mehrzelliger Tiere bestehen.

3. Tauchfähige und schwimmende Vorrichtung für die biologische Reinigung vor Ort von Netzen oder Geflechten, die zur Fischzucht verwendet werden, nach Anspruch 2, **gekennzeichnet durch** die Tatsache, dass sich das biologische Strahlungsmittel (3) im Inneren des Haupthohlkörpers (5) befindet, wo eine Ultraviolettstrahlungskammer gebildet ist, die mit mehreren UV-Strahlungslampen (8) ausgestattet ist, deren Stärke und Anzahl von dem empfangenen Flüssigkeitsstrom und festen Abfall abhängig ist.

4. Tauchfähige und schwimmende Vorrichtung für die biologische Reinigung vor Ort von Netzen oder Geflechten, die zur Fischzucht verwendet werden, nach Anspruch 3, **gekennzeichnet durch** die Tatsache, dass die Innenwände des Haupthohlkörpers (5) Spiegel (9) enthalten, die die Erhöhung des Strahlungseffekts der UV-Strahlungslampen (8) ermöglichen.

5. Tauchfähige und schwimmende Vorrichtung für die biologische Reinigung vor Ort von Netzen oder Geflechten, die zur Fischzucht verwendet werden, nach Anspruch 4, **gekennzeichnet durch** die Tatsache, dass sowohl die Spiegel (9) wie auch die UV-Strahlungslampen (8) an der Innenseite des Haupthohlkörpers (5) montiert sind, der seinerseits von der Hauptstruktur gestützt wird.

## Revendications

1. Dispositif submersible et flottant destiné au nettoyage biologique sur site de filets ou treillis utilisés pour l'élevage de poissons, qui permet la destruction des micro-organismes de l'eau et qui, en même temps, désinfecte et détruit les bactéries, champignons, virus et maladies des poissons qui pourraient autrement se propager sur l'environnement d'élevage, comportant des moyens (1) de soutien, un moyen de filtration de particules et des moyens de désinfection basés sur un rayonnement biologique ultraviolet, **caractérisé en ce que** les moyens (1) de soutien sont constitués d'une structure en métal, en plastique ou en bois, comprenant des moyens (4) de flottabilité qui maintiennent la structure (1) de soutien à flot et un corps principal creux (5) à l'intérieur duquel se trouvent les moyens de filtration de particules qui sont constitués d'une pluralité de sacs formés de filets en losange entourés sur les côtés et le fond par une ralingue en polypropylène.

2. Dispositif submersible et flottant destiné au nettoyage biologique sur site de filets ou treillis utilisés pour l'élevage de poissons, selon la revendication 1, **caractérisé en ce que** les sacs formés de filets en losange sont constitués de diverses mailles en losange dont les ouvertures présentent des diamètres de plus en plus petits jusqu'à atteindre une maille du diamètre d'ouverture minimal de façon à retenir et à contenir efficacement toutes les particules grossières de contaminant, généralement constituées d'algues et de restes d'animaux multicellulaires.

3. Dispositif submersible et flottant destiné au nettoyage biologique sur site de filets ou treillis utilisés pour l'élevage de poissons, selon la revendication 2, **caractérisé en ce que** le moyen (3) de rayonnement biologique se trouve à l'intérieur du corps principal creux (5) où est formée une chambre à rayonnement ultraviolet équipée d'une pluralité de lampes (8) à rayonnement UV dont la puissance et le nombre dépendent du débit de liquide et de déchets solides reçu.

4. Dispositif submersible et flottant destiné au nettoyage biologique sur site de filets ou treillis utilisés pour l'élevage de poissons, selon la revendication 3, **caractérisé en ce que** les parois intérieures du corps principal creux (5) comprennent des miroirs (9) qui permettent de renforcer l'effet du rayonnement desdites lampes (8) à rayonnement UV.

5. Dispositif submersible et flottant destiné au nettoyage biologique sur site de filets ou treillis utilisés pour l'élevage de poissons, selon la revendication 4, **caractérisé en ce qu'**à la fois les miroirs (9) et les lampes (8) à rayonnement UV sont montés à l'intérieur du corps principal creux (5) qui est lui-même soutenu par la structure principale.
